# EUROPÄISCHE PATENTSCHRIFT

(11) **EP 2 776 103 B1**
(45) Veröffentlichungstag und Bekanntmachung des Hinweises auf die Patenterteilung: **08.08.2018**
(21) Anmeldenummer: 11781548.0
(22) Anmeldetag: 11.11.2011
(51) Int. Cl.: A61M 16/08, A61M 16/04

(54) **VORRICHTUNG ZUR TRANSTRACHEALEN VENTILIERUNG**
TRANSTRACHEAL VENTILATION DEVICE
DISPOSITIF DESTINÉ À LA VENTILATION TRANSTRACHÉALE

(43) Veröffentlichungstag der Anmeldung: 17.09.2014
(73) Patentinhaber: Ventinova Technologies B.V., 5612 AR Eindhoven (NL)
(72) Erfinder: VAN ASSELDONK, Dirk Theodorus Andreas, NL-5464 PV Veghel (NL); HOGERWERF, Mark, NL-3465 JP Driebruggen (NL)
(74) Vertreter: Rössler, Matthias
(86) Internationale Anmeldenummer: PCT/EP2011/069948
(87) Internationale Veröffentlichungsnummer: WO 2013/068047

(56) Entgegenhaltungen:
- WO-A1-2011/038951
- DE-A1-102009 013 205
- GB-A- 2 137 506
- US-A- 3 987 798
- US-A- 4 235 229
- US-A- 4 869 718
- US-A- 5 778 877
- B. S. VADODARIA ET AL: "Comparison of four different emergency airway access equipment sets on a human patient simulator", ANAESTHESIA, Bd. 59, Nr. 1, 1. Januar 2004 (2004-01-01) , Seiten 73-79, XP55033443, ISSN: 0003-2409, DOI: 10.1111/j.1365-2044.2004.03456.x in der Anmeldung erwähnt

## Beschreibung

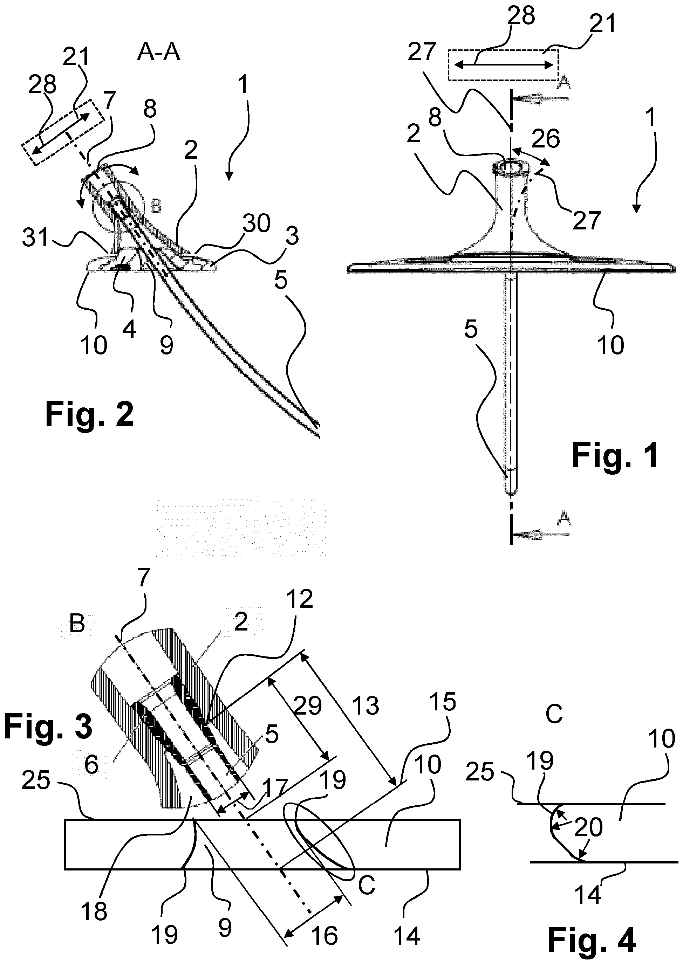

Die vorliegende Erfindung ist auf eine Vorrichtung zur transtrachealen Ventilierung von menschlichen Patienten gerichtet. Es sind verschiedene Vorrichtungen zur transtrachealen Ventilierung bekannt, z. B. aus dem Artikel "Comparison of four different emergency airway access equipment sets on a human patient Simulator" (Anaesthesia, 2004, 59, Seiten 73 bis 97, Figur 4). Dabei sind die bekannten Vorrichtungen zur transtrachealen Ventilierung jedoch nur für Patienten mit vergleichsweise normaler Anatomie geeignet.

Die vorliegende Erfindung hat sich nun zum Ziel gesetzt, den Anwendungsbereich der transtrachealen Ventilierung zu erweitern und insbesondere Patienten mit anomaler Anatomie, Schwellungen irgendeiner Art, Blutungen oder Ähnlichem, eine Vorrichtung zur Verfügung zu stellen, die an derartige Anomalitäten flexibel anpassbar ist. Es soll insbesondere erreicht werden, dass ein durch die Vorrichtung in den Patienten eingeführtes Lumen im Bereich der Vorrichtung nicht beschädigt wird und/oder knickt. Zudem soll die vorliegende Erfindung ein Bewegen des Patienten möglich machen, ohne dass die Vorrichtung verrutscht oder dass durch eine eventuelle Belastung, infolge der Bewegung des Patienten oder der Bewegung der Vorrichtung, dem Patienten Schmerzen oder sogar Verletzungen zugefügt werden.

Aufgabe der vorliegenden Erfindung ist daher das Bereitstellen einer Vorrichtung, die die Beatmung eines Patienten mittels transtrachealer Ventilation auch bei anomalen anatomischen Verhältnissen des Patienten ermöglicht und eine Beschädigung eines Lumens im Bereich der Vorrichtung verhindert sowie die Bewegungen des Patienten oder der Vorrichtung kompensiert.

Zur Lösung diese Aufgaben dient eine Vorrichtung nach Patentanspruch 1. Weitere vorteilhafte Ausgestaltungen sind in den abhängig formulierten Patentansprüchen angegeben. Es ist darauf hinzuweisen, dass die in den Patentansprüchen einzeln aufgeführten Merkmale in beliebiger, technologisch sinnvoller Weise miteinander kombiniert werden können, und weitere Ausgestaltungen der Erfindung aufzeigen. Die Beschreibung, insbesondere im Zusammenhang mit den Figuren, veranschaulicht weitere Ausführungsbeispiele der Erfindung.

Die Vorrichtung zur transtrachealen Ventilation umfasst zumindest eine Grundplatte mit einer Öffnung und ein schlauchartiges Anschlussteil mit einem Kanal und einer geometrischen Mittelachse. Die Grundplatte und das Anschlussteil sind so miteinander verbunden, dass ein Lumen sich durch die Öffnung in den Kanal entlang der Mittelachse erstreckt hin zu einer Befestigung, mit der das Lumen mit dem Anschlussteil verbindbar ist. Die Befestigung ist relativ zu der Mittelachse verschwenkbar. Die Befestigung ist in einem Abstand entlang der Mittelachse von mindestens 5 mm [Millimeter] von einer der Befestigung abgewandten ersten Seite der Grundplatte angeordnet und die Öffnung weist zumindest in einer zur Mittelachse senkrechten ersten Ebene einen Innendurchmesser auf, der zumindest um 20 % größer ist als ein Außendurchmesser des Lumens.

Aus der DE 10 2009 013 205, die hiermit vollumfänglich in Bezug genommen wird, ist ein Katheter bekannt zur Beatmung eines Patienten, der hier entweder durch das Lumen hindurchgeführt werden kann oder das Lumen ersetzt. Die US4235229 offenbart eine Vorrichtung zur transtrachealen Ventilation. Insbesondere ist das Anschlussteil (zumindest teilweise) aus einem flexiblen Material gefertigt, so dass es relativ zur Grundplatte in Bezug zur Mittelachse, insbesondere in allen Dimensionen, elastisch biegbar ist. Insbesondere ist ein Biegewinkel von 20°, insbesondere 30° und in besonders vorteilhafter Weise von 45° im Bezug zur Mittelachse (und insbesondere in zwei Richtungen ausgehend von der Mittelachse) möglich, ohne dass derartige Kräfte auf die Grundplatte wirken, dass der Patient infolge der Biegung des Anschlussteils belastet wird.

Insbesondere ist das Anschlussteil aus einem biegesteifen Material gefertigt und schwenkbar an der Grundplatte angeordnet. Insbesondere ist das Anschlussteil in mindestens einer zweiten Ebene, bevorzugt in nur einer zweiten Ebene, verschwenkbar. Insbesondere ist ein Biegewinkel von 20°, insbesondere 30° und in besonders vorteilhafter Weise von 45° im Bezug zur Mittelachse (und insbesondere in beide Richtungen ausgehend von der Mittelachse) möglich. Die mindestens eine zweite Ebene verläuft insbesondere in derselben Ebene wie die Luftröhre des Patienten. Die verschwenkte Stellung des Anschlussteils (ausgehend von einer Grundstellung) wird insbesondere auch ohne äußere Krafteinwirkung beibehalten.

Die verschwenkbare Anordnung des Anschlussteils ermöglicht eine flexible Anordnung des Lumens bzw. eines Katheters in Bezug auf den Patienten. Der Winkel, unter dem das Lumen bzw. der Katheter in den Patienten eingeführt wird, ist durch die verschwenkbare Anordung des Anschlussteils nun anpassbar an ggf. vorliegende Anomalitäten des Patienten.

Insbesondere ist durch das offene Ende des Anschlussteils ein Lumen einführbar zur Führung eines Katheters, der in den Patienten eingebracht werden kann. In Inneren des Katheters verläuft ein Beatmungskanal zur Zu- und Abfuhr von Sauerstoff und oder Luft. Das Lumen ist insbesondere schlauchartig und flexibel. Das Lumen wird insbesondere durch die Luftröhre des Patienten, z. B. über eine Punktierungsstelle am Hals des Patienten, in den Patienten eingeführt. Das Lumen verläuft entlang der Mittelachse durch das Anschlussteil. Das Anschlussteil umschließt an der Grundplatte die Öffnung, durch die das Lumen sich erstreckt. Das Lumen ist an einer Befestigung mit dem Anschlussteil verbindbar, insbesondere kraftschlüssig und/oder formschlüssig. Die Öffnung bildet entsprechend einen Durchlass für das Lumen/den Katheter durch die Grundplatte. Dieser Durchlass erstreckt sich von der der Befestigung zugewandten zweiten Seite der Grundplatte bis zu der von der Befestigung abgewandten ersten Seite der Grundplatte.

Der Abstand zwischen Befestigung und der von der Befestigung abgewandten ersten Seite der Grundfläche wird entlang der Mittelachse ermittelt. Die Befestigung endet hin zur Grundplatte insbesondere in einer Ebene senkrecht zur Mittelachse. Der Schnittpunkt dieser Ebene mit der Mittelachse definiert gemeinsam mit dem Schnittpunkt zwischen Mittelachse und der Ebene der abgewandten ersten Seite der Grundplatte den Abstand zwischen Befestigung und der abgewandten ersten Seite der Grundplatte. Damit kann der Abstand insbesondere unabhängig von der Neigung des Anschlussteils bzw. des Lumens gegenüber der, durch die abgewandte ersten Seite der Grundplatte gebildeten Ebene ermittelt werden. Insbesondere liegt die Vorrichtung mit der von der Befestigung abgewandten ersten Seite der Grundplatte auf dem Patienten auf. Der Schnittpunkt zwischen der Mittelachse und der Ebene der abgewandten ersten Seite der Grundplatte ist insbesondere deckungsgleich mit der Punktierungstelle am Patienten, an der das Lumen und/oder ein Katheter in den Patienten eintritt.

Das Lumen wird insbesondere im Bereich des Schnittpunkts zwischen Mittelachse und der Ebene der abgewandten ersten Seite der Grundplatte in den Patienten eingeführt. Durch den Abstand von mindestens 5 mm wird sichergestellt, dass das Anschlussteil mit dem Lumen relativ zur Mittelachse verschwenkt werden kann, ohne dass dem Patienten Schmerzen oder sogar Verletzungen zugefügt werden. Weiterhin wird ein Abknicken des Lumens im Bereich der ersten Seite der Grundplatte vermieden. Durch ein entsprechendes Verschwenken des Anschlussteils (und damit des Lumens) kann sichergestellt werden, dass das Lumen mit einem möglichst großen Biegeradius in den Patienten eingeführt wird.

Das Lumen ist in der Vorrichtung insbesondere ausschließlich über die Befestigung mit der Vorrichtung verbunden. Die Öffnung in der Grundplatte dient lediglich zur Durchführung des Lumens und bietet einen Freiraum gegenüber dem Außendurchmesser des Lumens. Somit kann die Vorrichtung auf dem Hals des Patienten verrutschen, ohne dass dem Patienten infolge einer resultierenden Bewegung des Lumens Schmerzen oder sogar Verletzungen zugeführt werden. Durch das Spiel zwischen Öffnung und Lumen wird ebenfalls erreicht, dass das Lumen mit einem möglichst großen Biegeradius in den Patienten eingeführt wird. Ein Abknicken des Lumens wird vermieden. Weiterhin wird durch das Spiel zwischen Öffnung und Lumen und durch die Verschwenkbarkeit des Anschlussteils der Winkel, unter dem das Lumen/der Katheter in den Patienten eingeführt wird, anpassbar an ggf. vorliegende Anomalitäten des Patienten.

Die Öffnung weist in einer zur Mittelachse senkrechten ersten Ebene einen Innendurchmesser auf, der zumindest um 20 % größer ist als ein Außendurchmesser des Lumens. Die Öffnung ist insbesondere nicht auf eine kreisrunde Ausführung beschränkt. Der Begriff Innendurchmesser bezieht sich hier lediglich auf das kleinste Maß der Öffnung, durch die das Lumen hindurchgeführt wird. Auch das Lumen ist entsprechend nicht auf eine kreisrunde Form beschränkt. Der Außendurchmesser gibt entweder das größte Querschnittsmaß des Lumens an oder, bei festgelegter Orientierung zwischen Lumen und Öffnung, das für jede Orientierung jeweils festgelegte Querschnittsmaß. Entsprechend sind insbesondere die jeweiligen, einander zugeordneten, Werte für Innendurchmesser der Öffnung und Außendurchmesser des Lumens miteinander zu vergleichen.

Die Grundstellung der Mittelachse ist durch die unbelastete Anordnung der Vorrichtung oder durch eine Mittelstellung definiert. Gegenüber dieser Grundstellung kann eine Verschwenkung des Anschlussteils bzw. der Befestigung gegenüber der Grundplatte erfolgen.

Gemäß einer besonders vorteilhaften Ausgestaltung der Vorrichtung beträgt der Abstand mindestens 8 mm, bevorzugt mindestens 12 mm und besonders bevorzugt mindestens 18 mm.

Gemäß einer weiteren besonders vorteilhaften Ausgestaltung ist die Öffnung durch mindestens eine Kante begrenzt, wobei die mindestens eine Kante einen Kantenradius von mindestens 1 mm aufweist. Durch die abgerundeten Kanten im Bereich der Öffnung kann eine Verletzung oder Beschädigung des Lumens weitgehend ausgeschlossen werden. Entsprechend sind bevorzugt sämtliche, die Öffnung begrenzenden Kanten mit einem entsprechenden, ggf. unterschiedlichen Kantenradius auszuführen. Eine abgerundete Kante verringert das Risiko des Abknickens des Lumens.

Insbesondere ist die Öffnung konusartig ausgeführt, wobei die kleinere Öffnung an der dem Anschlussteil zugewandten zweiten Seite der Grundplatte angeordnet ist und entsprechend die größere Öffnung an der dem Anschlussteil bzw. der Befestigung abgewandten ersten Seite der Grundplatte. Insbesondere weist die Öffnung in einer Ebene, die parallel zu der von der Befestigung abgewandten ersten Seite der Grundplatte verläuft, einen rechteckigen oder elliptischen Querschnitt auf, wobei das größte Maß der Öffnung um mindestens 30 % größer ist als das kleinste Maß der Öffnung.

Insbesondere ist die Befestigung in einer Distanz von mindestens 1 Millimeter, bevorzugt mindestens 2,5 Millimeter von der der Befestigung zugewandten zweiten Seite der Grundplatte beabstandet angeordnet. Die Distanz wird entlang der Mittelachse ermittelt. Die Befestigung endet hin zur Grundplatte insbesondere in einer Ebene senkrecht zur Mittelachse. Der Schnittpunkt dieser Ebene mit der Mittelachse definiert gemeinsam mit dem Schnittpunkt zwischen Mittelachse und der, im Bereich der Öffnung vorliegenden Ebene der der Befestigung zugewandten zweiten Seite der Grundplatte die Distanz zwischen Befestigung und der zugewandten zweiten Seite der Grundplatte. Damit kann die Distanz insbesondere unabhängig von der Neigung des Anschlussteils bzw. des Lumens gegenüber der, durch die ersten Seite der Grundplatte gebildeten Ebene ermittelt werden.

Gemäß einer weiteren besonders vorteilhaften Ausgestaltung ist die Befestigung in mindestens oder genau einer zweiten Ebene, die parallel zur Mittelachse verläuft, um mindestens 20° [Winkelgrad], bevorzugt um mindestens 30°, besonders bevorzugt um mindestens 45° zu der Mittelachse (und insbesondere in beide Richtungen ausgehend von der Mittelachse) verschwenkbar. Insbesondere ist die Befestigung in allen Ebenen, die parallel zur Mittelachse verlaufen (und durch die sich die Mittelachse erstreckt), um mindestens 20°, bevorzugt um mindestens 30°, besonders bevorzugt um mindestens 45° zu der Mittelachse verschwenkbar.

Gemäß einer weiteren besonders vorteilhaften Ausgestaltung der Vorrichtung ist das Anschlussteil lösbar mit der Grundplatte verbunden. Eine lösbare Verbindung bedeutet insbesondere, dass hier keine Zerstörung von (allen) Komponenten der Vorrichtung erforderlich ist, um das Anschlussteil von der Grundplatte zu trennen.

Damit kann die Vorrichtung kostengünstig eingesetzt werden, da eine Reinigung einzelner Komponenten möglich ist und bei entsprechender Beschädigung einzelner Komponenten der Austausch dieser einzelnen Komponenten ebenfalls möglich ist.

Gemäß einer weiteren Ausgestaltung ist das Anschlussteil unlösbar mit der Grundplatte verbunden, so dass eine besonders haltbare Vorrichtung geschaffen ist. Dabei können das Anschlussteil und die Grundplatte z.B. durch Zwei-Komponenten-Spritzgießen hergestellt werden oder durch einen Kleber miteinander verbunden werden.

Gemäß einer vorteilhaften Weiterbildung weist das Anschlussteil mindestens einen Flansch auf, der sich in die Grundplatte hinein erstreckt, wobei der mindestens eine Flansch durch eine formschlüssige Verbindung mit der Grundplatte verbindbar ist.

Insbesondere ist der mindestens eine Flansch durch jeweils (mindestens) ein Fixierungselement an der Grundplatte fixiert.

Bevorzugt ist die Grundplatte zumindest durch ein Flanschteil und ein Mittelteil gebildet. Insbesondere sind das Mittelteil und das Anschlussteil an aneinander gegenüberliegenden Seiten das Flanschteils so angeordnet, dass das Flanschteil gehalten ist durch eine lösbare Verbindung zwischen dem Anschlussteil und dem Mittelteil.

Insbesondere weist das Mittelteil eine Aufnahme und/oder ein Gegenstück zu dem mindestens einen Flansch des Anschlussteils auf, so dass der mindestens eine Flansch mit dem Mittelteil verbindbar ist. Insbesondere kommt mindestens ein Fixierungselement zum Einsatz, durch das der mindestens eine Flansch an dem Mittelteil fixiert wird. Diese Verbindung ist insbesondere lösbar, so dass eine zerstörungsfreie Trennung zumindest der Komponenten Grundplatte, Flanschteil, Mittelteil und Anschlussteil möglich ist.

Bevorzugt sind Flanschteil und Mittelteil unlösbar zu der Grundplatte miteinander verbunden, so dass lediglich eine zerstörungsfreie Trennung der Komponenten Grundplatte und Anschlussteil möglich ist. Flanschteil und Mittelteil können z.B. durch Zwei-Komponenten-Spritzgießen hergestellt werden, so dass in einem Herstellungsverfahren eine Grundplatte aus zwei verschiedenen Materialien mit jeweils unterschiedlichen Materialeigenschaften hergestellt werden kann. Flanschteil und Mittelteil sind insbesondere durch einen Klebprozess und/oder einen Schmelzprozess unlösbar miteinander verbunden.

Insbesondere ist das Flanschteil verformbar ausgeführt. Dadurch kann das Flanschteil sich flexibel an den Patienten anpassen. Insbesondere ist das Mittelteil biegesteifer als das Flanschteil oder sogar biegesteif ausgeführt. Somit kann eine Anbindung des Anschlussteils an das Mittelteil erfolgen und eine entsprechend steife, aber verschwenkbare Positionierung des Anschlussteils gegenüber dem Mittelteil bzw. gegenüber dem Patienten gewährleistet werden.

Gemäß einer bevorzugten Ausgestaltung besteht das Anschlusseil (zumindest teilweise) aus einem elastisch biegbaren/verformbaren Material.

Gemäß einer anderen bevorzugten Ausgestaltung besteht das Anschlusseil aus einem biegesteifen Material.

Insbesondere ist zwischen Anschlussteil und Grundplatte zumindest ein erster Spalt und/oder ein zweiter Spalt vorgesehen ist, der ein Verschwenken des Anschlussteils gegenüber der Grundplatte ermöglicht und im Wesentlichen einen Biegewinkel beschränkt. Insbesondere bei Verschwenken des biegesteifen Anschlussteils wird durch den ersten und oder zweiten Spalt ein Biegewinkel festgelegt, um den das Anschlussteil aus der Grundstellung gegenüber der Grundplatte verschwenkt werden kann. Die entsprechenden, den ersten und/oder zweiten Spalt begrenzenden Oberflächen des Anschlussteils und der Grundplatte bilden Anschläge, so dass ein weiteres Verschwenken nicht möglich ist.

Gemäß einer weiteren Ausgestaltung der Vorrichtung ist das offene Ende des Anschlussteils als weiblicher Teil einer Luer-Lock Verbindung ausgeführt. Insbesondere ist am offenen Ende eine Positionierungshilfe vorgesehen, so dass ein (gebogener) Katheter mit einer vorgegebenen Orientierung in der Vorrichtung angeordnet werden kann.

Insbesondere sind zwei Flansche an dem Anschlussteil vorgesehen, die sich in jeweils mindestens eine entsprechende Aufnahme des Mittelteils hinein erstrecken. Durch die Aufnahme und die jeweiligen Flansche hindurch wird insbesondere jeweils ein Stift als Fixierungselement eingesetzt, so dass Flansche und Mittelteil miteinander formschlüssig verbunden sind. Bei dieser Anordnung ist auch eine Verschwenkung um die Fixierungselemente möglich, so dass hierdurch neben der Flexibilität des Anschlussteils selbst die Verschwenkung der Befestigung gegenüber der Grundplatte ermöglicht wird. Es ist insbesondere möglich diese Verschwenkung durch die Ausführung des Anschlussteils aus flexiblem Material zu unterstützen. Das Anschlussteil kann aber auch aus einem biegesteifen Material gefertigt sein, das eine Biegung/Verformung des Anschlussteils selbst verhindert. In diesem Fall wird die Verschwenkung des Anschlussteils nur durch die schwenkbare Anordnung des Anschlussteils an der Grundplatte ermöglicht.

Insbesondere wird der mindestens eine Flansch durch ein Gewinde gebildet, das in ein Gegengewinde im Mittelteil eingreift. Damit wird eine formschlüssige Verbindung zwischen dem mindestens einen Flansch und dem Mittelteil, bzw. zwischen Anschlussteil und Mittelteil ausgebildet.

Es wird ausdrücklich festgestellt, dass die auf die Verbindung von Anschlussteil mit der Grundplatte gerichteten Merkmale auch unabhängig von der erfindungsgemäßen Vorrichtung verfolgt werden können. In diesem Fall umfasst die Vorrichtung zur transtrachealen Ventilation zumindest eine Grundplatte mit einer Öffnung und ein schlauchartiges Anschlussteil mit einem Kanal und einer Mittelachse. Die Grundplatte und das Anschlussteil sind so miteinander verbunden, dass ein Lumen sich durch die Öffnung in den Kanal entlang der Mittelachse erstreckt hin zu einer Befestigung, mit der das Lumen mit dem Anschlussteil verbindbar ist. Das Anschlussteil ist insbesondere lösbar und/oder formschlüssig mit der Grundplatte verbunden. Insbesondere ist die Befestigung relativ zu der Mittelachse verschwenkbar. Das Anschlussteil kann aus biegesteifem oder elastisch verformbarem Material hergestellt sein.

Weiter Einzelheiten der Erfindung sowie bevorzugte Ausführungsbeispiele werden im Folgenden anhand der Figuren erläutert, ohne dass die Erfindung auf die dort gezeigten Ausführungsbeispiele beschränkt wäre. Es zeigen schematisch:
- Fig. 1:: eine Vorrichtung zur transtrachealen Ventilation;
- Fig. 2:: die Vorrichtung gemäß Fig. 1 in einer Seitenansicht;
- Fig. 3:: ein Detail aus Fig. 2;
- Fig. 4:: ein Detail aus Fig. 3;
- Fig. 5:: eine perspektivische Ansicht der Vorrichtung; und
- Fig. 6:: eine Explosionszeichnung der Vorrichtung.

Fig. 1 zeigt schematisch eine erfindungsgemäße Vorrichtung 1 zur transtrachealen Ventilation, die eine Grundplatte 10 umfasst. Die Grundplatte 10 weist Aussparungen zur Befestigung von Fixierungsbändern auf. Diese dienen der Fixierung der Vorrichtung 1 an einem Patienten. Auf der Grundplatte 10 ist ein (flexibles) verschwenkbares Anschlussteil 2 angeordnet, das sich in Richtung weg von der Grundplatte 10 und dem Patienten entlang einer Mittelachse 7 erstreckt und ein offenes Ende 8 aufweist. Das Anschlussteil 2 ist insbesondere aus einem flexiblen Material gefertigt, so dass es relativ zur Grundplatte 10 in Bezug zur Mittelachse 7 insbesondere in alle Dimensionen biegbar/verschwenkbar ist. Insbesondere ist ein Biegewinkel 26 von 20° [Winkelgrad], insbesondere 30° und in besonders vorteilhafter Weise 45° zur Mittelachse 7 möglich, ohne dass derartige Kräfte auf die Grundplatte 10 wirken, dass der Patient aufgrund der Verschwenkung belastet wird. Durch das offene Ende 8 des Anschlussteils 2 ist ein Lumen 5 einführbar zur Führung eines Katheters, der in den Patienten eingebracht werden soll. Im Inneren des Katheters verläuft ein Beatmungskanal zur Luftzu- und abfuhr von Sauerstoff und/oder Luft. Das Lumen 5 ist schlauchartig und flexibel und wird durch die Luftröhre des Patienten z. B. über eine Punktierungsstelle am Hals des Patienten eingeführt. Das Lumen 5 wird entlang der Mittelachse 7 durch das offene Ende 8 in das Anschlussteil 2 eingeführt. Das Anschlussteil 2 umschließt an der Grundplatte 10 die Öffnung 9, durch die das Lumen 5 sich erstreckt. In Fig. 1 ist weiterhin die zweite Ebene 21 gezeigt, in der das Anschlussteil 2 gegenüber der Grundplatte bzw. der Grundstellung 27 der Mittelachse 7 verschwenkbar ist. Die Ebene 21 (hier angedeutet durch das gestrichelte Rechteck) wird durch die Mittelachse 7 und den Vektor 28 aufgespannt.

Fig. 2 zeigt in einer Seitenansicht die Vorrichtung 1 gemäß Fig. 1 im Schnitt A-A. Die Grundplatte 10 teilt sich in ein Flanschteil 3 und ein Mittelteil 4 auf, wobei das Mittelteil 4 eine Öffnung 9 aufweist zur Durchführung des Lumens 5. Die Grundplatte 10 weist mit ihrer Unterseite zum Patienten, so dass das Lumen 5 über das offene Ende 8 des Anschlussteils 2 und durch die Öffnung 9 in den Patienten einführbar ist. Das Anschlussteil 2 ist insbesondere flexibel ausgeführt und in einer zweiten Ebene 21 (hier angedeutet durch das gestrichelte Rechteck) gegenüber der Grundstellung 27 der Mittelachse 7 um einen Biegewinkel 26 biegbar/verschwenkbar. Auch hier wird die Ebene 21 durch die Mittelachse 7 und den Vektor 28 aufgespannt. Bevorzugt ist das Anschlussteil 2 aus einem biegesteifem Material hergestellt, so dass es lediglich in der hier in Fig. 2 gezeigten zweiten Ebene 21 gegenüber der Grundstellung 27 der Mittelachse 7 um einen Biegewinkel 26 verschwenkbar ist. Der Biegewinkel 26 erstreckt sich ausgehend von der Mittelachse 7 in beide möglichen Richtungen in der zweiten Ebene 21. Die zweite Ebene 21 in Fig. 2 weist eine andere Orientierung auf als in Fig. 1. Die zweite Ebene 21 in Fig. 2 ist parallel zu der Ebene, in der die Luftröhre des Patienten verläuft.

Das Anschlussteil 2 ist insbesondere in verschiedenen zweiten Ebenen 21 verschwenkbar. Das Lumen 5 erstreckt sich bis in die Nähe des offenen Endes 8 des Anschlussteils 2 und ist dort in einer Befestigung 12 befestigt (siehe Detail B in Fig. 3). Das Lumen 5 kann sich aber auch über das offene Ende 8 hinaus erstrecken und über die Befestigung 12 mit dem Anschlussteil 2 verbunden sein. Durch die Aufteilung der Grundplatte 10 in Flanschteil 3 und Mittelteil 4 ist die Grundplatte 10 auch aus verschiedenen Materialien, z. B. durch Zweikomponenten-Spritzgießen, oder in einzelnen voneinander getrennten Komponenten herstellbar und somit an spezielle Anforderungen anpassbar.

Zwischen Anschlussteil 2 und Grundplatte 10 sind ein erster Spalt 30 und ein zweiter Spalt vorgesehen, die die Verschwenkung des (biegesteifen) Anschlussteils 2 gegenüber der Grundplatte 10 ermöglichen. Gleichzeitig wird durch die Größe des ersten Spaltes 30 und des zweiten Spaltes 31 der mögliche Biegewinkel 26, also der Grad der Verschwenkung gegenüber der Grundstellung 27 definiert und beschränkt.

Fig. 3 zeigt das Detail B gemäß Fig. 2, wobei das Lumen 5 sich in das hohle Anschlussteil 2 hinein erstreckt und durch eine Hülse 6 zwischen der Wandung des Anschlussteils 2 und der Hülse 6 an eine Befestigung 12 geklemmt wird. Für diese Klemmung ist das Anschlussteil 2 speziell mit einer Aufnahme ausgestattet, so dass eine sichere Fixierung des Lumens 5 durch die Befestigung 12 möglich ist und ein Durchrutschen des Lumens 5 in Richtung der Grundplatte 10 bzw. in Richtung des Patienten verhindert wird. Insbesondere ist das Anschlussteil 2 am offenen Ende 8 als Luer-Lock ausgeführt. Dabei ist die an dem Anschlussteil 2 vorgesehene Verbindungsseite als weibliche Verbindung der Luer-Lock Verbindung ausgeführt. Entlang der Mittelachse 7 erstreckt sich der Kanal 18 durch das Anschlussteil 2 und durch die Öffnung 9 in der Grundplatte 10. In diesen Kanal 18 erstreckt sich nun das Lumen 5 ausgehend von der Befestigung 12 hin zu der Öffnung 9. Das Lumen 5 weist einen Außendurchmesser 17 auf. Die Öffnung 9 weist in einer zur Mittelachse 7 senkrecht angeordneten ersten Ebene 15 einen Innendurchmesser 16 auf. Dieser Innendurchmesser 16 ist durch Kanten 19 begrenzt. Die Öffnung 9 erweitert sich, ausgehend von einer hin zur Befestigung 12 orientierten zweiten Seite 25 der Grundplatte 10, hin zur anderen, gegenüber der Befestigung 12 abgewandten ersten Seite 14 der Grundplatte 10. Der Innendurchmesser 16 der Öffnung 9 weist ein Spiel gegenüber dem Außendurchmesser 17 des Lumens 5 auf, so dass bei einer Verschwenkung des Anschlussteils 2 und damit des Lumens 5 eine Kontaktierung von Lumen 5 und einer Kante 19 der Öffnung 9 vermieden wird.

Die Befestigung 12 ist gegenüber diesem Schnittpunkt bzw. gegenüber der der Befestigung abgewandten ersten Seite 14 der Grundplatte 10 in einem Abstand 13 angeordnet. Durch diesen Abstand 13 wird gewährleistet, dass eine Verschwenkung der Befestigung 12, also des Lumens 5, im Anschlussteil 2 möglich ist, so dass ein möglichst großer Biegeradius des Lumens 5 im Bereich der Vorrichtung 1 erhalten wird. Ein Abknicken des Lumens 5, insbesondere durch Kontaktierung mit den Kanten, wird entsprechend verhindert. Die Befestigung 12 ist gegenüber der der Befestigung 12 zugewandten zweiten Seite 25 der Grundplatte 10 in einer Distanz 29 angeordnet.

Fig. 4 zeigt ein Detail C aus Fig. 3. Dabei ist hier eine Kante 19 der Öffnung 9 in der Grundplatte 10 gezeigt, die durch einen Kantenradius 20 definiert ist. Damit wird eine scharfkantige Kante 19 vermieden, durch die das Lumen 5 infolge Kontaktierung mit der Kante 19 verletzt werden könnte. Die Öffnung 9 erstreckt sich von der zweiten Seite 25 der Grundplatte 10 hin zu der ersten Seite 14. Die einzelnen Kanten 19 weisen insbesondere unterschiedliche Kantenradien 20 auf.

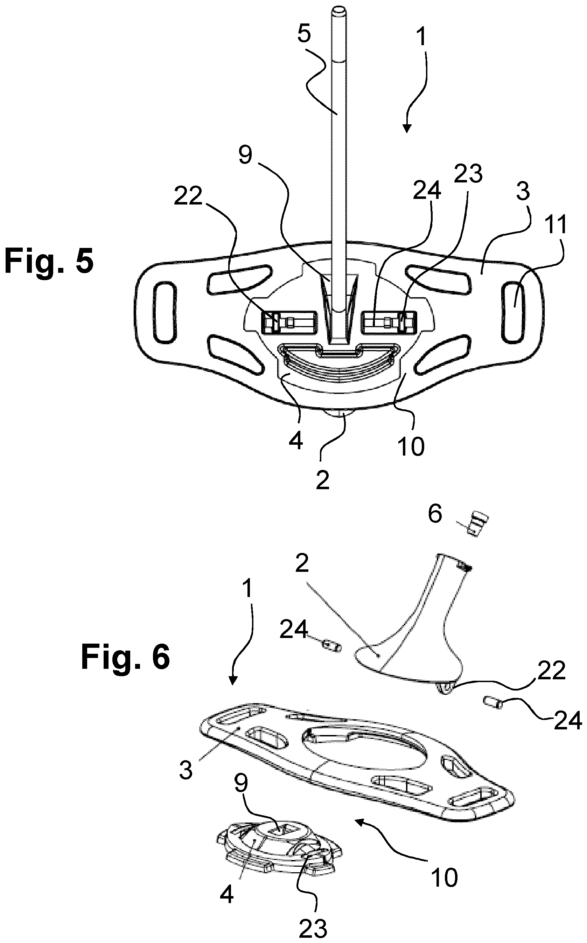

Fig. 5 zeigt in einer perspektivischen Ansicht die Unterseite der Vorrichtung 1 bzw. der Grundplatte 10. Hier ist gezeigt, dass die Grundplatte 10 aus einem Flanschteil 3 und einem Mittelteil 4 besteht, wobei das Mittelteil 4 die Öffnung 9 zur Durchführung des Lumens 5 aufweist. Das Flanschteil 3 weist mehrere Aussparungen 11 auf, wobei die außen gelegenen Aussparungen 11 rechts und links zur Befestigung eines Halsgurtes geeignet sind zur Fixierung der Vorrichtung 1 an dem Patienten. Die weiteren vier Aussparungen 11, die um das Mittelteil 4 in dem Flanschteil 3 angeordnet sind, dienen zur Fixierung der Vorrichtung 1 an dem Patienten. In dem Mittelteil 4 ist die sich hin zum Patienten erweiternde Öffnung 9 gezeigt, durch die sich das Lumen 5 ausgehend von dem Anschlussteil 2 erstreckt. Das Anschlussteil 2 weist zwei Flansche 22 (hier lediglich einer sichtbar) auf, die sich in die Grundplatte 10 hinein erstrecken und über Fixierungselemente 24 eine Verbindung 23 mit dem Mittelteil 4 ausbilden. Dadurch ist eine lösbare Verbindung 23 zwischen Mittelteil 4 und Anschlussteil 2 gewährleistet. Es können insbesondere weitere Klemmelemente vorgesehen sein, die die Fixierungselemente 24 dauerhaft in Position halten. Diese Klemmelemente können eine zerstörungsfreie Trennung der Komponenten Flansch 22 und Mittelteil ggf. verhindern.

Fig. 6 zeigt in einer Explosionsdarstellung die Vorrichtung 1, wobei Mittelteil 4 und Flanschteil 3 zu der Grundplatte 10 zusammengefügt werden. Das flexible Anschlussteil 2 wird an der Grundplatte 10 angeordnet.

Die Hülse 6 dient der Fixierung des Lumens 5 in dem Anschlussteil 2 an der Befestigung 12. Das Mittelteil 4 weist eine sich hin zum Patienten erweiternde Öffnung 9 auf und Öffnungen zur Gestaltung einer Verbindung 23 mit den Flanschen 22 des Anschlussteils 2. Dazu sind Stifte als Fixierungselemente 24 hier vorgesehen, die sich durch die Flansche 22 und durch jeweils eine entsprechende Aufnahme im Mittelteil 4 hindurch erstrecken. Durch diese Fixierungselemente 24 wird eine Verbindung 23 zwischen Mittelteil 4 und Anschlussteil 2 erzeugt, so dass das Anschlussteil 2 an der Grundplatte 10 (schwenkbar) fixiert wird. Die Fixierungselemente 24 werden erst nach der gemeinsamen Anordnung von Anschlussteil 2, Flanschteil 3 und Mittelteil 4 zur Erzeugung der Verbindung 23 im Mittelteil angeordnet. Durch den Einsatz der Stifte als Fixierungselemente 24 ist eine (zusätzliche) Schwenkbewegung des Anschlussteils 2 gegenüber der Grundplatte 10 möglich, wobei in diesem Fall die gemeinsame Achse der Stifte die Schwenkachse des Anschlussteils 2 bildet.

### Bezugszeichenliste

- 1: Vorrichtung
- 2: Anschlussteil
- 3: Flanschteil
- 4: Mittelteil
- 5: Lumen
- 6: Hülse
- 7: Mittelachse
- 8: Ende
- 9: Öffnung
- 10: Grundplatte
- 11: Aussparung
- 12: Befestigung
- 13: Abstand
- 14: erste Seite
- 15: erste Ebene
- 16: Innendurchmesser
- 17: Außendurchmesser
- 18: Kanal
- 19: Kante
- 20: Kantenradius
- 21: Zweite Ebene
- 22: Flansch
- 23: Verbindung
- 24: Fixierungselement
- 25: zweite Seite
- 26: Biegewinkel
- 27: Grundstellung
- 28: Vektor
- 29: Distanz
- 30: Erster Spalt
- 31: Zweiter Spalt

## Patentansprüche

1. Vorrichtung (1) zur transtrachealen Ventilation, mit zumindest einer Grundplatte (10) mit einer Öffnung (9) und einem schlauchartigen Anschlussteil (2) mit einem Kanal (18) und einer Mittelachse (7), wobei die Grundplatte (10) und das Anschlussteil (2) so miteinander verbunden sind, dass ein Lumen (5) sich durch die Öffnung (9) in den Kanal (18) entlang der Mittelachse (7) erstreckt hin zu einer Befestigung (12), mit der das Lumen (5) mit dem Anschlussteil (2) verbindbar ist, wobei die Befestigung (12) relativ zu der Mittelachse (7) verschwenkbar ist, wobei die Befestigung (12) in einem Abstand (13) entlang der Mittelachse (7) von mindestens 5 mm [Millimeter] von einer der Befestigung (12) abgewandten ersten Seite (14) der Grundplatte (10) angeordnet ist und die Öffnung (9) zumindest in einer zur Mittelachse (7) senkrechten ersten Ebene (15) einen Innendurchmesser (16) aufweist, der zumindest um 20 % größer ist als ein Außendurchmesser (17) des Lumens (5); **dadurch gekennzeichnet dass** die Befestigung (12) in zumindest einer zweiten Ebene (21), die parallel zur Mittelachse (7) verläuft, um mindestens 20° [Winkelgrad] zu der Mittelachse (7) verschwenkbar ist.

2. Vorrichtung (1) nach Patentanspruch 1, wobei der Abstand (13) mindestens 8 mm, bevorzugt mindestens 12 mm und besonders bevorzugt mindestens 18 mm beträgt.

3. Vorrichtung (1) nach einem der vorhergehenden Patentansprüche, wobei die Öffnung (9) durch mindestens eine Kante (19) begrenzt ist, wobei die mindestens eine Kante (19) einen Kantenradius (20) von mindestens 1 mm aufweist.

4. Vorrichtung (1) nach einem der vorhergehenden Patentansprüche, wobei das Anschlussteil (2) lösbar mit der Grundplatte (10) verbunden ist.

5. Vorrichtung (1) nach einem der vorhergehenden Patentansprüche, wobei das Anschlussteil (2) mindestens einen Flansch (22) aufweist, der sich in die Grundplatte (10) hinein erstreckt, wobei der Flansch (22) durch eine formschlüssige Verbindung (23) mit der Grundplatte (10) verbindbar ist.

6. Vorrichtung (1) nach Patentanspruch 5, wobei der mindestens eine Flansch (22) durch jeweils ein Fixierungselement (24) an der Grundplatte (10) fixiert ist.

7. Vorrichtung (1) nach einem der vorhergehenden Patentansprüche, wobei die Grundplatte (10) zumindest durch ein Flanschteil (3) und ein Mittelteil (4) gebildet ist, wobei das Mittelteil (4) und das Anschlussteil (2) an aneinander gegenüberliegenden Seiten (14, 25) des Flanschteils (3) so angeordnet sind, dass das Flanschteil (3) gehalten ist durch eine lösbare Verbindung zwischen dem Anschlussteil (2) und dem Mittelteil (4).

8. Vorrichtung (1) nach einem der vorhergehenden Patentansprüche, wobei die Grundplatte (10) zumindest durch ein Flanschteil (3) und ein Mittelteil (4) gebildet ist, wobei das Mittelteil (4) aus einem biegesteiferen Material hergestellt ist als das Flanschteil (3).

9. Vorrichtung (1) nach einem der vorhergehenden Patentansprüche, wobei die Grundplatte (10) zumindest durch ein Flanschteil (3) und ein Mittelteil (4) gebildet ist, wobei die Grundplatte durch ein Zwei-Komponenten-Spritzgießverfahren hergestellt ist.

10. Vorrichtung (1) nach einem der vorhergehenden Patentansprüche, wobei zwischen Anschlussteil (2) und Grundplatte (10) zumindest ein erster Spalt (30) und/oder ein zweiter Spalt (31) vorgesehen ist, der ein Verschwenken des Anschlussteils (2) gegenüber der Grundplatte (10) ermöglicht und im Wesentlichen einen Biegewinkel (26) beschränkt.

11. Vorrichtung (1) nach einem der vorhergehenden Patentansprüche, wobei das Anschlüssen (2) aus einem elastisch biegbaren Material besteht.

12. Vorrichtung (1) nach einem der vorhergehenden Patentansprüche 1 bis 10, wobei das Anschlussteil (2) aus einem biegesteifen Material besteht.

## Claims

1. Device (1) for transtracheal ventilation, having at least a base plate (10) with an opening (9), and a tubular connecting part (2) with a channel (18) and a central axis (7), wherein the base plate (10) and the connecting part (2) are connected to each other such that a lumen (5) extends through the opening (9) into the channel (18) along the central axis (7) toward a fastening (12), by which the lumen (5) can be connected to the connecting part (2), wherein the fastening (12) is pivotable relative to the central axis (7), wherein the fastening (12) is arranged at a distance (13) of at least 5 mm [millimeters] along the central axis (7) from a first side (14) of the base plate (10) directed away from the fastening (12), and the opening (9), at least in a first plane (15) perpendicular to the central axis (7), has an internal diameter (16) which is at least 20% greater than an external diameter (17) of the lumen (5), **characterized in that** the fastening (12), in at least a second plane (21) extending parallel to the central axis (7), is pivotable through at least 20° [angle degrees] to the central axis (7).

2. Device (1) as claimed in patent claim 1, wherein the distance (13) is at least 8 mm, preferably at least 12 mm, and particularly preferably at least 18 mm.

3. Device (1) as claimed in one of the preceding patent claims, wherein the opening (9) is delimited by at least one edge (19), wherein the at least one edge (19) has an edge radius (20) of at least 1 mm.

4. Device (1) as claimed in one of the preceding patent claims, wherein the connecting part (2) is connected releasably to the base plate (10).

5. Device (1) as claimed in one of the preceding patent claims, wherein the connecting part (2) has at least one flange (22) which extends into the base plate (10), wherein the flange (22) can be connected to the base plate (10) by a form-fit connection (23).

6. Device (1) as claimed in patent claim 5, wherein the at least one flange (22) is fixed on the base plate (10) by in each case a fixing element (24).

7. Device (1) as claimed in one of the preceding patent claims, wherein the base plate (10) is formed at least by a flange part (3) and a middle part (4), wherein the middle part (4) and the connecting part (2) are arranged on mutually opposite sides (14, 25) of the flange part (3), such that the flange part (3) is held by a releasable connection between the connecting part (2) and the middle part (4).

8. Device (1) as claimed in one of the preceding patent claims, wherein the base plate (10) is formed at least by a flange part (3) and a middle part (4), wherein the middle part (4) is made from a more flexurally stiff material than the flange part (3).

9. Device (1) as claimed in one of the preceding patent claims, wherein the base plate (10) is formed at least by a flange part (3) and a middle part (4), wherein the base plate is produced by a two-component injection-molding method.

10. Device (1) as claimed in one of the preceding patent claims, wherein at least a first gap (30) and/or a second gap (31) is provided between connecting part (2) and base plate (10), which gap permits a pivoting of the connecting part (2) relative to the base plate (10) and substantially limits a bend angle (26).

11. Device (1) as claimed in one of the preceding patent claims, wherein the connecting part (2) is made from an elastically bendable material.

12. Device (1) as claimed in one of the preceding patent claims 1 through 10, wherein the connecting part (2) is made from a flexurally stiff material.

## Revendications

1. Dispositif (1) destiné à la ventilation transtrachéale, avec au moins une plaque de base (10) avec une ouverture (9) et une partie de raccordement (2) du type tuyau flexible avec un canal (18) et un axe central (7), dans lequel la plaque de base (10) et la partie de raccordement (2) sont assemblées l'une à l'autre de telle manière qu'une lumière (5) s'étende à travers l'ouverture (9) dans le canal (18) le long de l'axe central (7) en direction d'une fixation (12), avec laquelle la lumière (5) peut être raccordée à la partie de raccordement (2), dans lequel la fixation (12) peut pivoter par rapport à l'axe central (7), dans lequel la fixation (12) est disposée à une distance (13) le long de l'axe central (7) d'au moins 5 mm [millimètres] d'un côté de la plaque de base (10) détourné de la fixation (12) et l'ouverture (9) présente au moins dans un premier plan (15) perpendiculaire à l'axe central (7) un diamètre intérieur (16), qui est d'au moins 20 % plus grand qu'un diamètre extérieur (17) de la lumière (5), **caractérisé en ce que** la fixation (12) peut pivoter d'au moins 20° [degrés d'angle] par rapport à l'axe central (7) dans au moins un second plan (21), qui s'étend parallèlement à l'axe central (7).

2. Dispositif (1) selon la revendication 1, dans lequel la distance (13) vaut au moins 8 mm, de préférence au moins 12 mm et de préférence encore au moins 18 mm.

3. Dispositif (1) selon l'une quelconque des revendications précédentes, dans lequel l'ouverture (9) est limitée par au moins un bord (19), dans lequel ledit au moins un bord (19) présente un rayon de bord (20) d'au moins 1 mm.

4. Dispositif (1) selon l'une quelconque des revendications précédentes, dans lequel la partie de raccordement (2) est reliée de façon détachable à la plaque de base (10).

5. Dispositif (1) selon l'une quelconque des revendications précédentes, dans lequel la partie de raccordement (2) présente au moins une bride (22), qui s'étend vers l'intérieur de la plaque de base (10), dans lequel la bride (22) peut être assemblée à la plaque de base (10) par un assemblage à emboîtement (23).

6. Dispositif (1) selon la revendication 5, dans lequel ladite au moins une bride (22) est fixée à la plaque de base (10) respectivement par un élément de fixation (24).

7. Dispositif (1) selon l'une quelconque des revendications précédentes, dans lequel la plaque de base (10) est formée au moins par une partie de bride (3) et une partie médiane (4), dans lequel la partie médiane (4) et la partie de raccordement (2) sont disposées sur des côtés opposés l'un à l'autre (14, 25) de la partie de bride (3), de telle manière que la partie de bride (3) soit maintenue par un assemblage détachable entre la partie de raccordement (2) et la partie médiane (4).

8. Dispositif (1) selon l'une quelconque des revendications précédentes, dans lequel la plaque de base (10) est formée au moins par une partie de bride (3) et une partie médiane (4), dans lequel la partie médiane (4) est formée en un matériau plus rigide en flexion que la partie de bride (3).

9. Dispositif (1) selon l'une quelconque des revendications précédentes, dans lequel la plaque de base (10) est formée au moins par une partie de bride (3) et une partie médiane (4), dans lequel la plaque de base est fabriquée par un procédé de moulage par injection à deux composants.

10. Dispositif (1) selon l'une quelconque des revendications précédentes, dans lequel il est prévu entre la partie de raccordement (2) et la plaque de base (10) une première fente (30) et/ou une seconde fente (31), qui permet un pivotement de la partie de raccordement (2) par rapport à la plaque de base (10) et limite essentiellement un angle de flexion (26).

11. Dispositif (1) selon l'une quelconque des revendications précédentes, dans lequel la partie de raccordement (2) se compose d'un matériau élastiquement flexible.

12. Dispositif (1) selon l'une quelconque des revendications précédentes 1 à 10, dans lequel la partie de raccordement (2) se compose d'un matériau rigide en flexion.
